# EUROPEAN PATENT APPLICATION

(11) **EP 2 985 048 A1**
(43) Date of publication of application: **17.02.2016**
(21) Application number: 13881534.5
(22) Date of filing: 27.12.2013
(51) Int. Cl.: A61M 5/32

(54) **INJECTION NEEDLE**

(30) Priority: 08.04.2013 JP 2013080528
(71) Applicant: OLYMPUS CORPORATION, Shibuya-ku Tokyo 151-0072 (JP)
(72) Inventor: YOSHIDA, Kazuhiro, Tokyo 151-0072 (JP)
(74) Representative: Schicker, Silvia
(86) International application number: PCT/JP2013/085098
(87) International publication number: WO 2014/167756

(57) **Abstract**

An object is to easily insert a puncture needle into a pericardial cavity while reliably preventing a blade tip of the puncture needle from being brought into contact with the heart. Provided is a puncture needle (1) that includes: an elongated approximately-linear main body (2); a distal end portion (3) that is located at a distal end of the main body (2) and that has a blade surface (31) formed of a distal end surface which is formed inclined with respect to a longitudinal direction; and a bending portion (4) that is provided between the main body (2) and the distal end portion (3) to connect the main body (2) to the distal end portion (3) and that can be passively bent in response to an external force. The distal end portion (3) functions as a bending-direction control means for setting a bending direction of the bending portion (4) to a direction in which the blade surface (31) is made to face toward an inner side of a bent shape.

## Description

### {Technical Field}

The present invention relates to a puncture needle, particularly, to a puncture needle used to percutaneously insert a medical instrument, such as a guide wire, into the pericardial cavity from outside the body.

### {Background Art}

In the related art, a method in which a hollow needle is percutaneously inserted into a space (pericardial cavity) between the heart and the pericardium from outside the body, and a guide wire is inserted into the pericardial cavity through the needle is used in heart operations. In this method, an epidural-anesthesia puncture needle having a bent distal end portion is used (for example, see PTL 1).

When the pericardium is punctured by a needle, in order to prevent a blade tip thereof from being brought into contact with the heart, the pericardium is punctured by the needle at a shallow angle to the surface of the heart (an angle closer to a line parallel to the surface of the heart) while checking the positional relationship between the needle and the heart on an X transparent image etc. At this time, by using the needle having the bent distal end portion, it is possible to insert the needle into the pericardial cavity while making the blade tip face toward an opposite side from the heart and preventing the blade tip from being brought into contact with the heart.

On the other hand, there are known injection needles provided with a mechanism for changing a bending angle of the distal end portion in order to adjust the puncture direction (for example, see PTLs 2 and 3).

### {Citation List}

### {Patent Literature}

{PTL 1} Japanese Unexamined Patent Application, Publication No. 2009-95368
{PTL 2} Publication of Japanese Patent No. 4638276
{PTL 3} Publication of Japanese Patent No. 4133867

### {Summary of Invention}

### {Technical Problem}

However, in the needles disclosed in PTLs 1 to 3, the bending angle of the distal end portion is fixed or is changed manually by an operator. Therefore, it is difficult to instantly change the position of the blade tip with respect to the surface of the heart depending on the situation. Specifically, there is a problem in that, at the time of needle puncturing, a precise needle operation is required such that the puncture angle of the needle with respect to the surface of the heart becomes the optimum angle, which is sufficiently shallow, and, if the puncture angle of the needle becomes deeper than the optimum angle, it is difficult to reliably prevent the blade tip from being brought into contact with the heart.

The present invention has been made in view of the above-described circumstances, and an object thereof is to provide a puncture needle that can be easily inserted into a pericardial cavity while reliably preventing the blade tip from being brought into contact with the heart.

### {Solution to Problem}

In order to achieve the above-described object, the present invention provides the following solutions.

The present invention provides a puncture needle including: an elongated approximately-linear main body; a distal end portion that is located at a distal end of the main body and that has a blade surface formed of a distal end surface which is formed inclined with respect to a longitudinal direction; a bending portion that is provided between the main body and the distal end portion to connect the main body to the distal end portion and that can be passively bent in response to an external force; and a bending-direction control means that sets a bending direction of the bending portion to a direction in which the blade surface is made to face toward an inner side of a bent shape.

According to the present invention, after the distal end portion is inserted into the pericardial cavity with the blade surface facing toward an opposite side from the heart, when the distal end portion is brought into contact with the heart in the pericardial cavity, the bending portion is passively bent. At this time, because the bending portion is bent, by the bending-direction control means, in a direction in which the blade tip is located on the inner circumferential side of the bent shape, the blade surface is moved in such a direction as to be moved away from the heart. Specifically, in the pericardial cavity, the location and the angle of the blade surface with respect to the surface of the heart are instantly appropriately changed by the curving of the bending portion. Accordingly, it is possible to easily perform insertion into the pericardial cavity while reliably preventing the blade tip from being brought into contact with the heart.

In the above-described invention, the bending-direction control means may be configured when the distal end portion is bent in an arc-like manner with the blade surface facing toward the inner side.

By doing so, the bent distal end portion is easily brought into contact with surrounding tissues, on a lateral surface thereof at an outer circumferential side, which is the opposite side from the blade surface, thereby setting the bending direction of the bending portion to a direction in which the blade surface is made to face inward. Accordingly, because there is no need to provide the bending portion with a structure for setting the bending direction, the degree of freedom in the design of the bending portion can be increased.

In the above-described invention, the bending portion may have a first flexural stiffness in a bending direction in which the blade surface is made to face toward the inner side and a second flexural stiffness in a bending direction in which the blade surface is made to face toward an outer side; and the bending-direction control means may be configured when the first flexural stiffness is smaller than the second flexural stiffness.

By doing so, the bending direction of the bending portion is set to a more-easy-to-bend direction in which the blade surface is made to face inward. Accordingly, because there is no need to provide the distal end portion with a structure for setting the bending direction, the degree of freedom in the design of the distal end portion can be increased.

In the above-described invention, the bending portion may have notches that are formed on a lateral surface and that extend in a circumferential direction thereof; and the notches may be formed, in the circumferential direction of the bending portion, on the same side as a blade base of the blade surface.

By doing so, with a simple configuration, the first flexural stiffness of the bending portion can be made smaller than the second flexural stiffness thereof.

In the above-described invention, the bending portion may have notches that are formed on a lateral surface and that extend in a circumferential direction thereof; and the notches may be formed, in the circumferential direction of the bending portion, on the same side as a blade base of the blade surface and on the same side as a blade tip of the blade surface, and the notches that are formed on the same side as the blade base may have larger widths in the longitudinal direction than the notches that are formed on the same side as the blade tip.

By doing so, with a simple configuration, the first flexural stiffness of the bending portion can be made smaller than the second flexural stiffness thereof.

In the above-described invention, in the circumferential direction of the bending portion, a side of the bending portion that is the same side as a blade base of the blade surface may be made of a material having a higher flexibility than that of a side thereof that is the same side as a blade tip of the blade surface.

By doing so, with a simple configuration, the first flexural stiffness of the bending portion can be made smaller than the second flexural stiffness thereof.

In the above-described invention, the bending portion may include: an elastic member that is extendable in the longitudinal direction of the main body; and an extension restricting member that restricts extension of the elastic member, on the same side as a blade base of the blade surface in the circumferential direction of the elastic member.

By doing so, with a simple configuration, the first flexural stiffness of the bending portion can be made smaller than the second flexural stiffness thereof.

In the above-described invention, the bending portion may be formed of a cylindrical member and may have a thin-walled portion having a thinner wall thickness on the same side as a blade base of the blade surface in the circumferential direction than on the same side as a blade tip of the blade surface.

By doing so, with a simple configuration, the first flexural stiffness of the bending portion can be made smaller than the second flexural stiffness thereof.

In the above-described invention, the bending portion may be formed of a cylindrical member and may have spiral slits formed in the circumferential direction in a region that excludes the same side of a blade base of the blade surface.

By doing so, with a simple configuration, the first flexural stiffness of the bending portion can be made smaller than the second flexural stiffness thereof.

In the above-described invention, the main body, the bending portion, and the distal end portion may be formed of a cylindrical member that has, therein, a hole passing therethrough in the longitudinal direction; and the puncture needle may further include an approximately-linear supporting member that is inserted into the hole of the main body, the bending portion, and the distal end portion, which are communicated with each other in the longitudinal direction, and that has a larger stiffness than the bending portion.

By doing so, in a situation in which a larger stiffness is required for the bending portion, by inserting the supporting member into the hole, the bending portion can be strengthened such that the bending portion is not bent unintentionally.

In the above-described invention, a distal end portion of the supporting member may have a bent shape and may be capable of being elastically deformed into an approximately-linear shape.

By doing so, when the supporting member is rotated in the hole in the circumferential direction, the shape of the bending portion can be easily changed between an approximately linear shape and a bent shape, at a desired timing.

In the above-described invention, the puncture needle may further include a wire member whose distal end is connected to the distal end portion and that extends to the base end of the main body.

By doing so, when the base end of the wire member is pulled, the bending portion can be bent at a desired timing and at a desired angle.

### {Advantageous Effects of Invention}

According to the present invention, an advantageous effect is afforded in that it is possible to easily perform insertion into the pericardial cavity while reliably preventing the blade tip from being brought into contact with the heart.

### {Brief Description of Drawings}

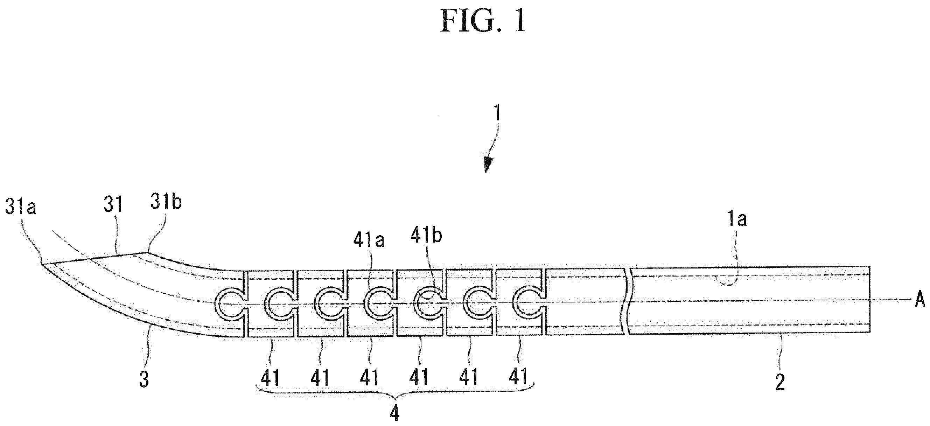

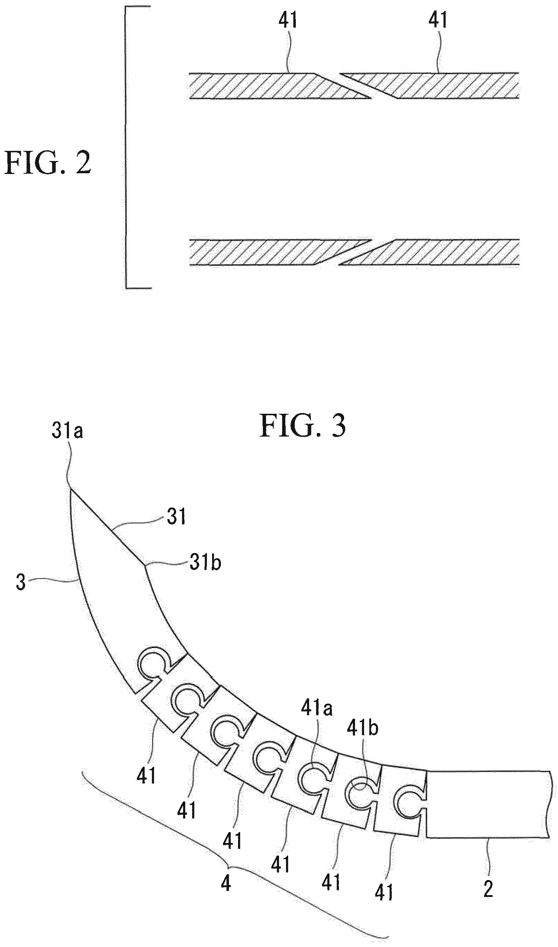

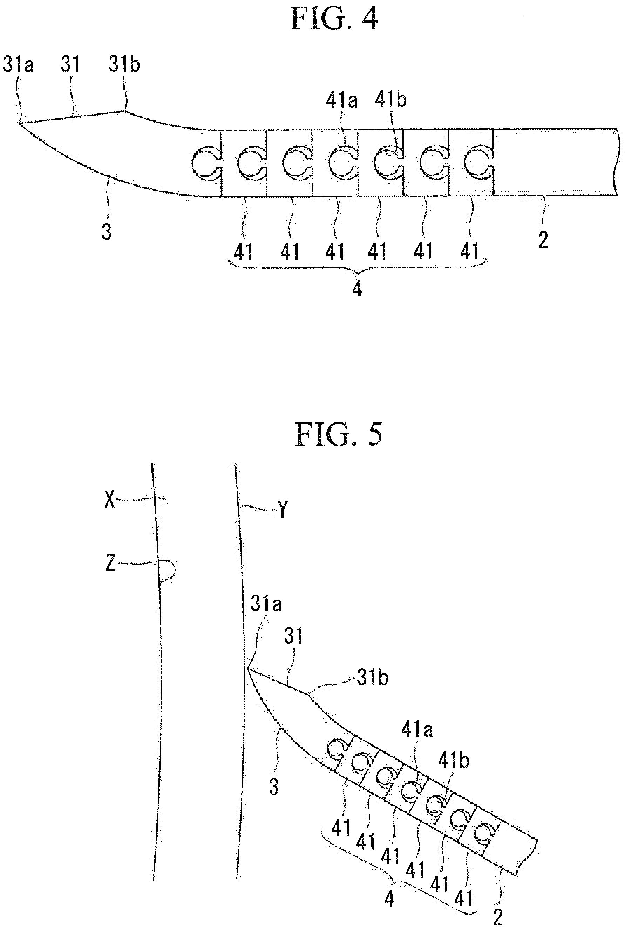

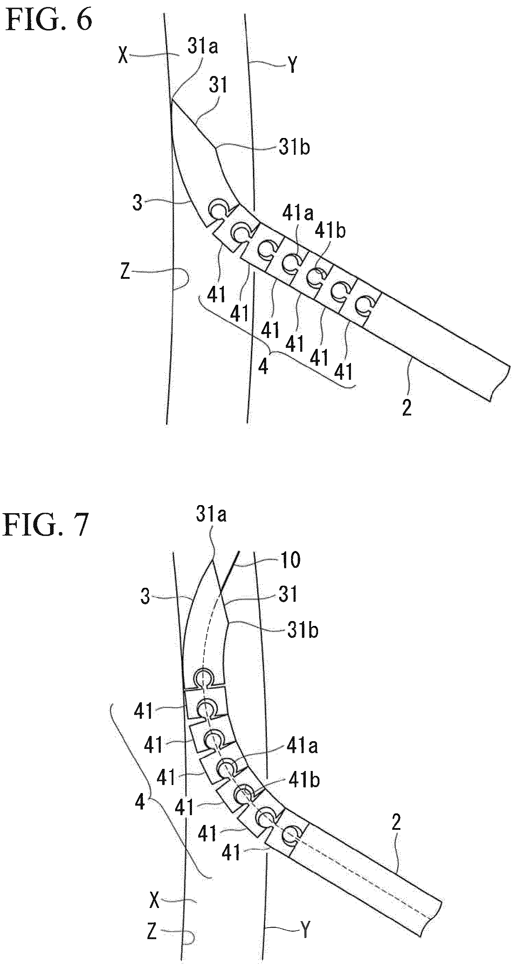

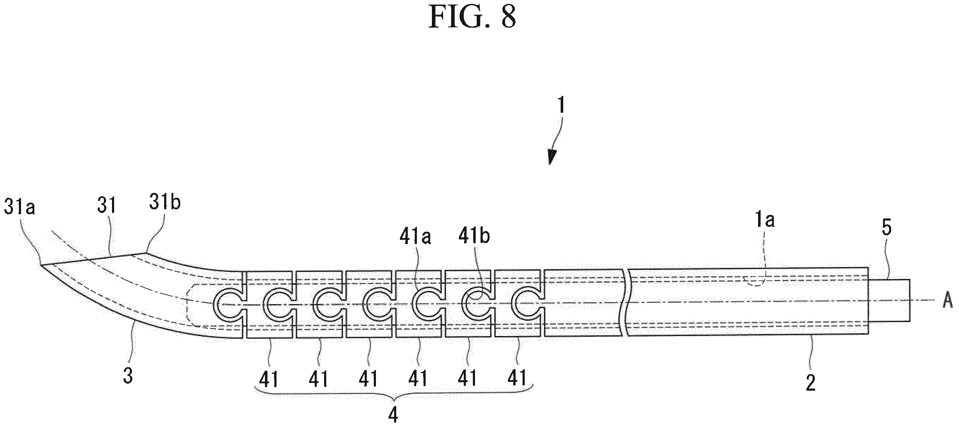

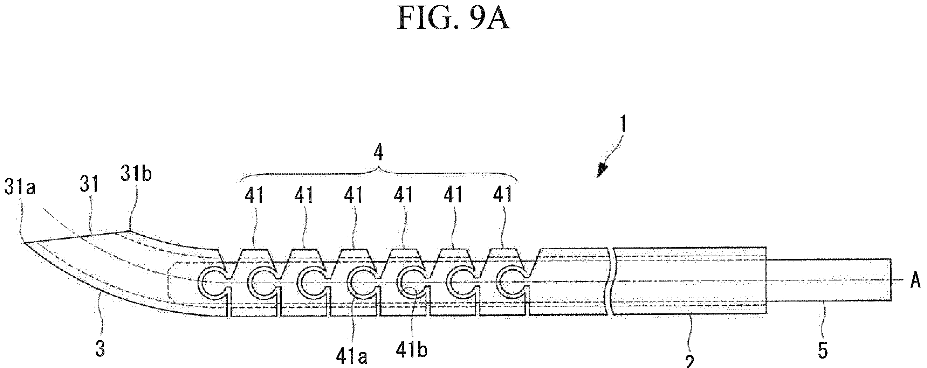

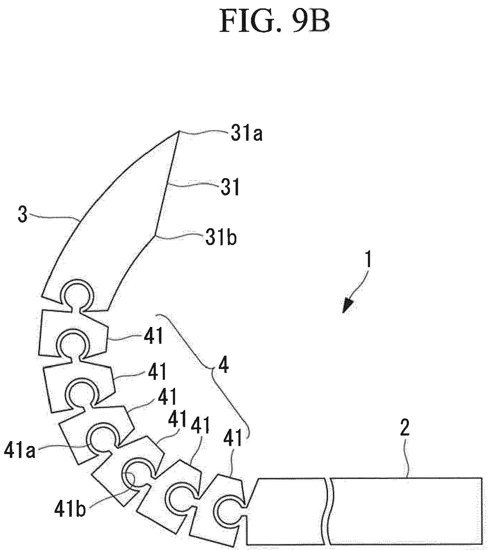

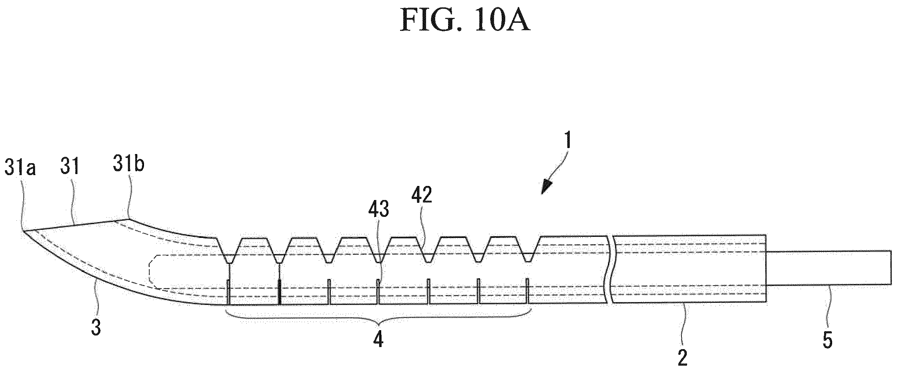

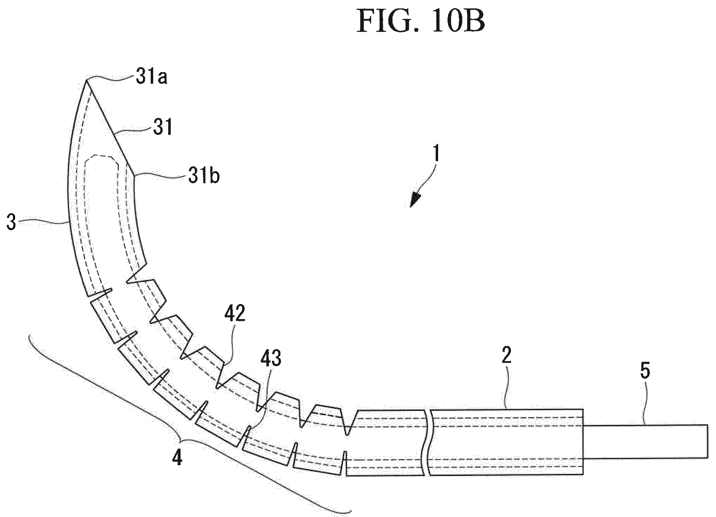

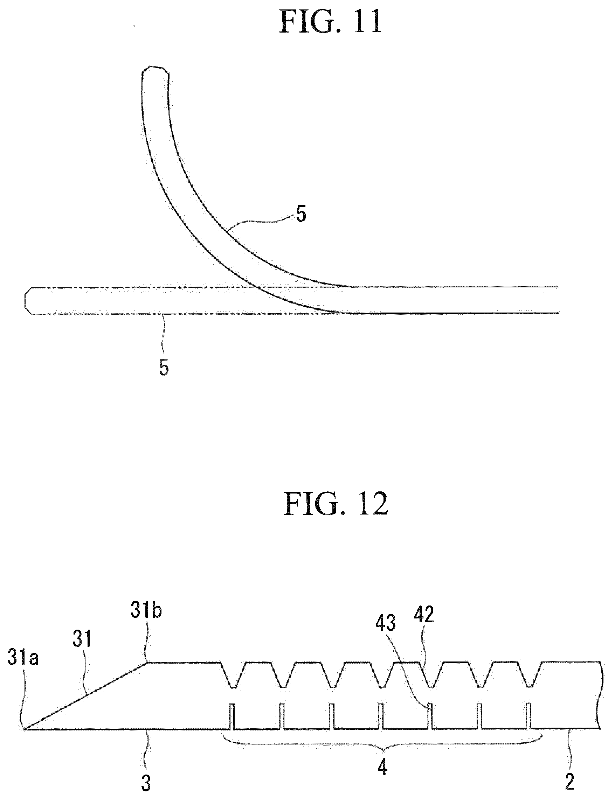

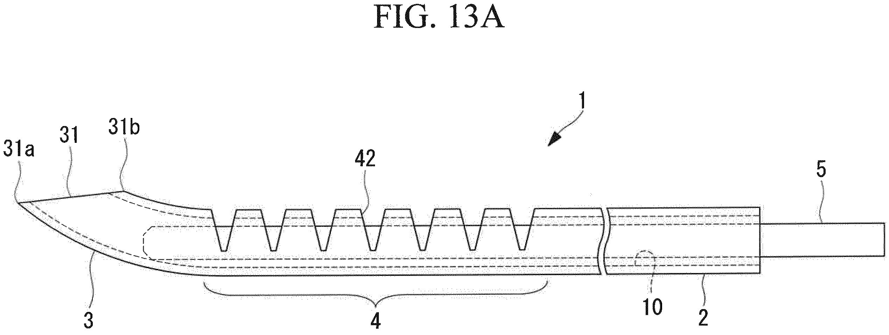

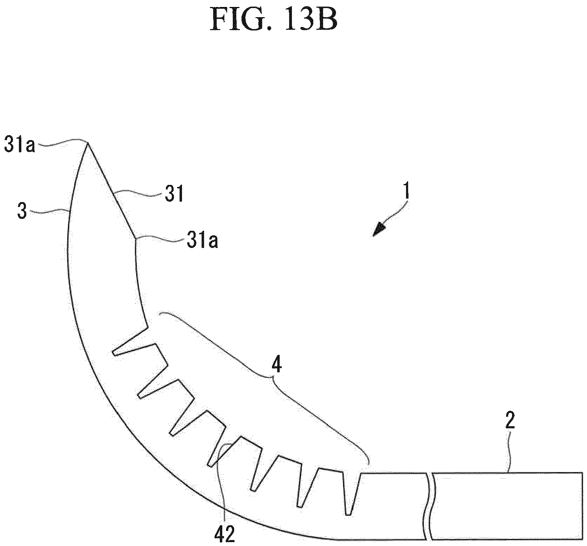

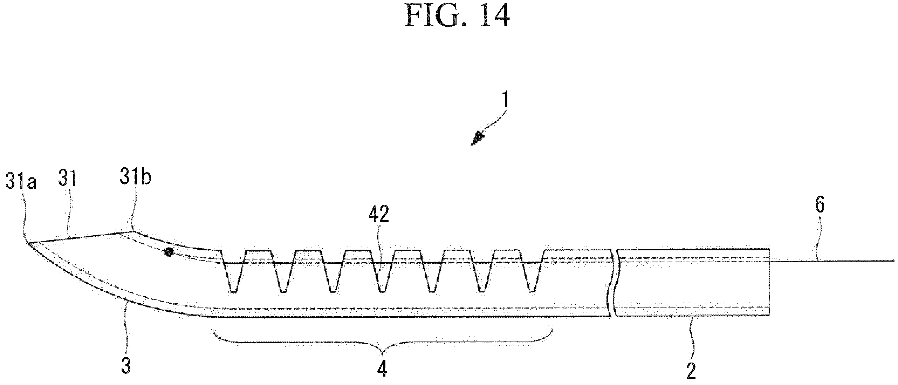

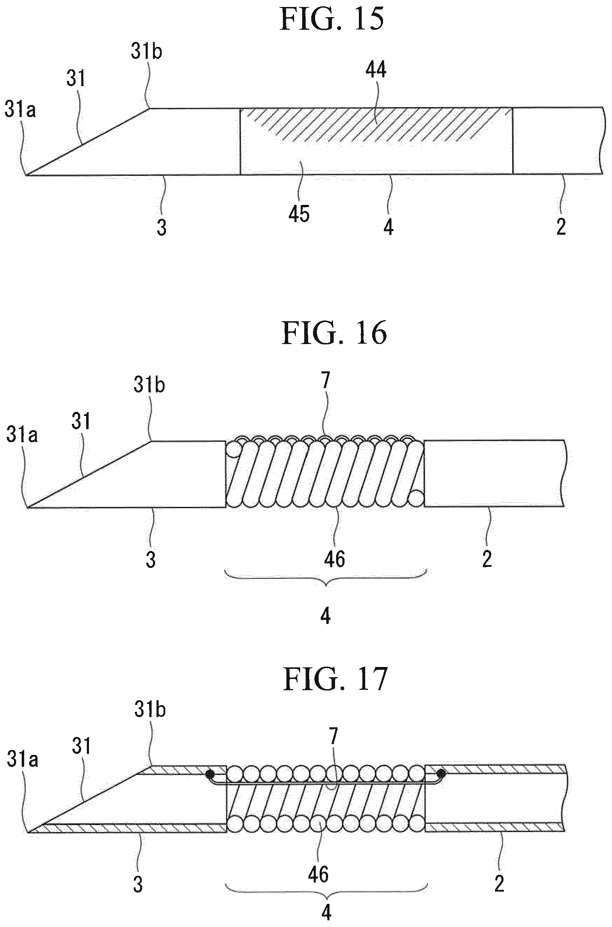

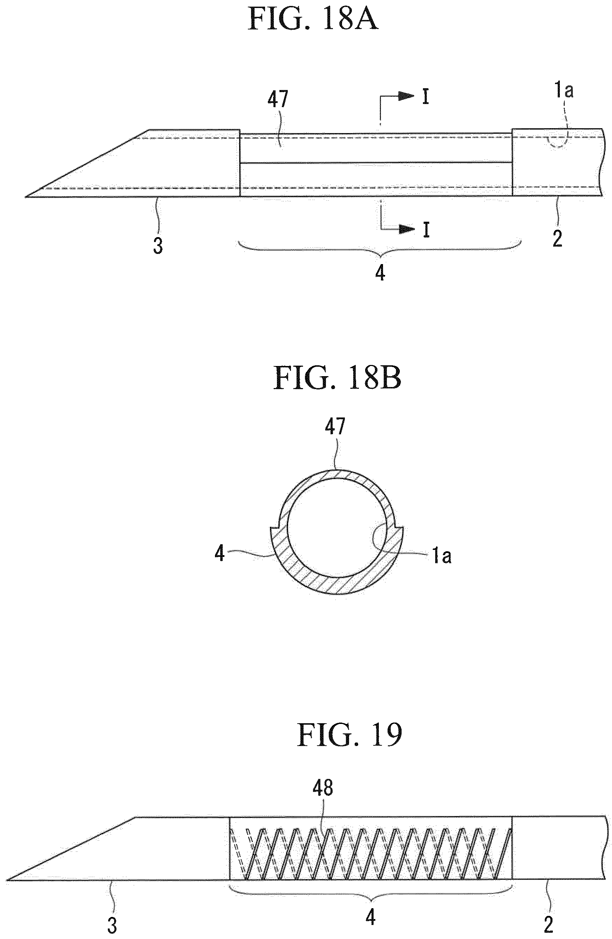

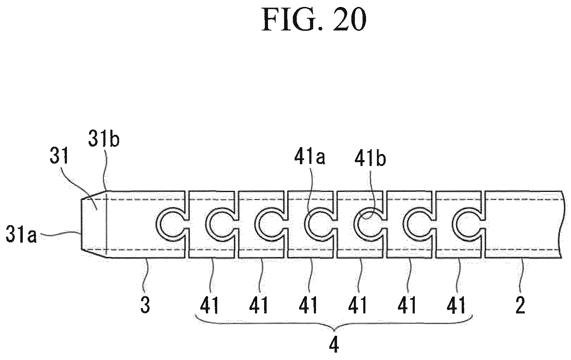

{Fig. 1} Fig. 1 is a view showing the overall configuration of a puncture needle according to one embodiment of the present invention.
{Fig. 2} Fig. 2 is a partial sectional view of a bending portion included in the puncture needle shown in Fig. 1.
{Fig. 3} Fig. 3 is a view showing a bent state of the bending portion included in the puncture needle shown in Fig. 1.
{Fig. 4} Fig. 4 is a view showing a rigid state of the bending portion included in the puncture needle shown in Fig. 1.
{Fig. 5} Fig. 5 is a view for explaining the operation of the puncture needle shown in Fig. 1.
{Fig. 6} Fig. 6 is a view for explaining the operation of the puncture needle shown in Fig. 1.
{Fig. 7} Fig. 7 is a view for explaining the operation of the puncture needle shown in Fig. 1.
{Fig. 8} Fig. 8 is a view showing the overall configuration of a modification of the puncture needle shown in Fig. 1, the modification including a supporting member.
{Fig. 9A} Fig. 9A is a view of the overall configuration of a puncture needle in a normal state, showing a bending portion and a bending-direction control means according to a first modification.
{Fig. 9B} Fig. 9B is a view of the overall configuration of the puncture needle in a bent state, showing the bending portion and the bending-direction control means according to the first modification.
{Fig. 10A} Fig. 10A is a view of the overall configuration of a puncture needle in a normal state, showing a bending portion and a bending-direction control means according to a second modification.
{Fig. 10B} Fig. 10B is a view of the overall configuration of the puncture needle in a bent state, showing the bending portion and the bending-direction control means according to the second modification.
{Fig. 11} Fig. 11 is a view for explaining the movement of a distal end portion of a supporting member included in the puncture needle shown in Fig. 10.
{Fig. 12} Fig. 12 is a view showing a partial configuration of a modification of the distal end portion included in the puncture needle shown in Fig. 10.
{Fig. 13A} Fig. 13A is a view of the overall configuration of a puncture needle in a normal state, showing a bending portion and a bending-direction control means according to a third modification.
{Fig. 13B} Fig. 13B is a view of the overall configuration of the puncture needle in a bent state, showing the bending portion and the bending-direction control means according to the third modification.
{Fig. 14} Fig. 14 is a view of the overall configuration of a modification of the puncture needle shown in Fig. 13, the modification including a wire.
{Fig. 15} Fig. 15 is a view of a partial configuration of a puncture needle, showing a bending portion and a bending-direction control means according to a fourth modification.
{Fig. 16} Fig. 16 is a view of a partial configuration of a puncture needle, showing a bending portion and a bending-direction control means according to a fifth modification.
{Fig. 17} Fig. 17 is a view of a partial configuration of a puncture needle, showing modifications of the bending portion and the bending-direction control means shown in Fig. 16.
{Fig. 18A} Fig. 18A is a view of a partial configuration of a puncture needle, showing a bending portion and a bending-direction control means according to a sixth modification.
{Fig. 18B} Fig. 18B is a sectional view along the line I-I in Fig. 18A, showing the bending portion and the bending-direction control means according to the sixth modification.
{Fig. 19} Fig. 19 is a view of a partial configuration of a puncture needle, showing a bending portion and a bending-direction control means according to a seventh modification.
{Fig. 20} Fig. 20 is a view of a partial configuration of a puncture needle, showing a modification of a blade surface.

### {Description of Embodiment}

A puncture needle 1 according to one embodiment of the present invention will be described below with reference to the drawings.

As shown in Fig. 1, the puncture needle 1 of this embodiment includes an approximately-linear elongated main body 2, a distal end portion 3 that is located at a distal end of the main body 2 and that is bent in an arc-like manner, and a bending portion 4 that is provided between the main body 2 and the distal end portion 3 to connect the main body 2 to the distal end portion 3.

The main body 2, the distal end portion 3, and the bending portion 4 are made from a metal having bioaffinity and an appropriate stiffness, such as stainless steel. Furthermore, the main body 2, the distal end portion 3, and the bending portion 4 form a cylindrical shape that is open at both ends, and the puncture needle 1 has a hole 1a that leads from a base end of the main body 2 to a distal end of the distal end portion 3 along a longitudinal central axis A (hereinafter, simply referred to as axis A) and into which a guide wire can be inserted.

The distal end portion 3 has a blade surface 31 formed of a distal end surface that is formed inclined with respect to the axis A. The distal end portion 3 is bent in the same direction as the blade surface 31, a blade tip 31a of the blade surface 31 is located at an outer circumferential side of the bent shape, a blade base 31b of the blade surface 31 is located at an inner side of the bent shape, and the blade surface 31 is made to face toward an inner side of the bent shape.

The bending portion 4 has a plurality of cylindrical bending segments 41 that are connected to each other in the axis-A direction. The adjacent bending segments 41 are connected to each other in a manner allowing them to swing about an axis in the direction perpendicular to the plane defined by the distal end portion 3 when bent in one direction (in the direction perpendicular to the plane of Fig. 1).

Specifically, each of the bending segments 41 has a circular axial portion 41a that is formed so as to protrude in the axis-A direction from one end and a circular notch portion 41b that is formed at the other end and into which the axial portion 41a of another adjacent bending segment 41 is fitted. Furthermore, as shown in Fig. 2, both ends of each bending segment 41 have shapes that allow them to radially engage with the ends of other adjacent bending segments 41.

In a normal state, the axial portion 41a is fitted into the inner periphery of the notch portion 41b, with some play therebetween. By using this play, each of the bending segments 41 can swing about the axial portion 41a of another adjacent bending segment 41. Accordingly, when an external force in a direction intersecting the axis A is applied to the distal end portion 3, the bending portion 4 is flexibly bent at a bending angle corresponding to this external force, as shown in Fig. 3.

On the other hand, when a pressing force in the axis-A direction is applied by bringing the distal end portion 3 into contact with an object, the adjacent bending segments 41 are brought into close contact in the axis-A direction, thereby making the bending portion 4 rigid in a linear manner, as shown in Fig. 4.

Note that, in the reference drawings, the reference signs 41a and 41b, which indicate the axial portion and the notch portion, are assigned to only one bending segment 41, in order to simplify the drawings.

Here, the distal end portion (bending-direction control means) 3 is easily brought into contact with surrounding objects, on a lateral surface thereof at an outer circumferential side of the bent shape. Accordingly, it is easy for the bending portion 4 to curve in the same direction as the bending direction of the distal end portion 3, i.e., in a direction in which the blade surface 31 is made to face toward an inner side of the bent shape, whereas it is difficult for the bending portion 4 to curve in the opposite direction to the bending direction of the distal end portion 3, i.e., in a direction in which the blade surface 31 is made to face toward an outer side of the bent shape.

Next, the operation of the thus-configured puncture needle 1 will be described by giving an example case in which a guide wire 10 is percutaneously inserted from outside the body into a pericardial cavity X.

In order to insert the guide wire 10 into the pericardial cavity X by using the puncture needle 1 of this embodiment, the puncture needle 1 is inserted into the body with the blade tip 31a facing forward and is moved forward to the vicinity of the pericardium Y while incising tissue with the blade tip 31a, as shown in Fig. 5.

While being moved forward in the tissue, the blade tip 31a is pressed by the tissue, thus putting the bending portion 4 into a rigid state with high stiffness (the state shown in Fig. 4); therefore, it is possible to easily incise the tissue with the blade tip 31a. Furthermore, at this time, the positional relationship between the puncture needle 1 and the heart Z is checked on an X-ray transparent image, for example, and the puncture needle 1 is located so as to be inclined with respect to the surface of the heart Z while making the blade surface 31 face toward an opposite side from the heart Z.

Next, the pericardium Y is punctured by the blade tip 31a to insert the distal end portion 3 into the pericardial cavity X. In the pericardial cavity X, because the distal end portion 3 is bent in such a direction as to form a convex shape with respect to the surface of the heart Z, as shown in Fig. 6, the blade tip 31a is made to point toward an opposite side from the surface of the heart Z. Here, when the distal end portion 3 enters the pericardial cavity X, the bending portion 4 is released from the pressing force applied by the tissue and is put into a flexible state in which it can be bent. Accordingly, in the pericardial cavity X, when the lateral surface on the outer circumferential side of the distal end portion 3 is brought into contact with the beating heart Z, the bending portion 4 is bent in such a direction as to move the blade surface 31 away from the heart Z, in response to the pressing force from the heart Z.

When the puncture needle 1 is moved farther forward, the bending portion 4 enters the pericardial cavity X while being flexibly bent, as shown in Fig. 7. Next, the guide wire 10 is inserted into the hole 1a from the base end of the main body 2, which is located outside the body, and the guide wire 10 is made to protrude from an opening in the blade surface 31. Next, while the position of the guide wire 10 is maintained, the puncture needle 1 is pulled out toward the base end along the guide wire 10, thereby making it possible to insert the guide wire 10 into the pericardial cavity X from outside the body.

In this way, according to this embodiment, because the bending portion 4 is passively bent in a direction in which the blade surface 31 is made to face inward, due to contact between the distal end portion 3 and the heart Z, when the distal end portion 3 is brought into contact with the heart Z, the blade tip 31a is moved in such a direction as to be moved away from the heart Z. Specifically, even if the puncture angle to the pericardium Y becomes relatively deep, in the pericardial cavity X, the angle of the blade surface 31 with respect to the surface of the heart Z is instantly changed to an appropriate angle, due to a change in the bending angle of the bending portion 4. Accordingly, it is possible to reliably prevent the blade tip 31a from being brought into contact with the heart Z and also to easily perform insertion of the puncture needle 1 into the pericardial cavity X by relaxing the accuracy of the puncture angle to the pericardium Y.

Note that, in this embodiment, in order to strengthen the stiffness of the bending portion 4 in a rigid state, an approximately-linear supporting member 5 that is inserted into the hole 1a when tissue is incised may be provided, as shown in Fig. 8.

By doing so, incision of the tissue by the blade tip 31a can be performed more easily. The supporting member 5 may be a member that has a larger flexural stiffness than the bending portion 4, and the guide wire 10 may be used as the supporting member 5.

Furthermore, in this embodiment, although the bending portion 4 has a plurality of bending segments 41, which are connected in the axis-A direction, and the bending direction of the arc-like distal end portion 3 sets the bending direction of the bending portion 4, the structure of the bending portion 4 and the bending-direction control means for setting the bending direction of the bending portion 4 are not limited thereto. Next, modifications of the puncture needle 1 of this embodiment that include bending portions 4 and bending-direction control means having different structures will be described with reference to Fig. 9A to Fig. 19.

### First Modification

In a puncture needle 1 according to a first modification of this embodiment, as shown in Fig. 9A, a gap between two adjacent bending segments 41 is formed wider on the same side as the blade base 31b in the circumferential direction (hereinafter, referred to as "inner circumferential side") than on the same side as the blade tip 31a in the circumferential direction (hereinafter, referred to as "outer circumferential side"). More preferably, the gap on the inner circumferential side is formed in a wedge shape whose width is gradually increased toward the radially outer side of the bending portion 4.

According to this modification, as shown in Fig. 9B, it is possible to further increase the maximum bending angle at which the bending portion 4 can be bent. In this modification, because the stiffness obtained in the rigid state is lower than that of the bending portion 4 shown in Fig. 1, additional use of the above-described supporting member 5 is preferable at the time of tissue incision.

### Second Modification

In a puncture needle 1 according to a second modification of this embodiment, as shown in Fig. 10A, the bending portion 4 is formed of a flexible cylindrical member, and first notches 42 and second notches 43 that extend in the circumferential direction are formed on the inner circumferential side and the outer circumferential side of the lateral surface, as bending-direction control means.

The second notches 43 have widths that are sufficiently narrow in the axis-A direction. On the other hand, the first notches 42 have widths that are sufficiently wider in the axis-A direction than those of the second notches 43. Accordingly, the bending portion 4 has a higher flexural stiffness in a bending direction in which the blade surface 31 is made to face inward than in a bending direction in which the blade surface 31 is made to face outward, and, as shown in Fig. 10B, the bending direction of the bending portion 4 is set to a bending direction in which the blade surface 31 is made to face inward. The first notches 42 are preferably formed in wedge shapes, like the gaps between the bending segments 41 of the first modification.

In this way, according to this modification, with a simple structure in which the notches 42 and 43, which have different widths, are provided on the lateral surface of the bending portion 4, the bending direction of the bending portion 4 can be set.

In this modification, additional use of the supporting member 5 is preferable in order to strengthen the stiffness of the bending portion 4. In this modification, as shown in Fig. 11, a distal end portion of the supporting member 5 has a bent shape in the normal state and can be elastically deformed into an approximately-linear shape, as indicated by a two-dot chain line in the figure.

When the supporting member 5 is inserted into the hole 1a with the distal end pointing toward the blade tip 31a, as shown in Fig. 10A, the distal end portion of the supporting member 5 is straightened in the hole 1a, thus straightening the bending portion 4. From this state, when the supporting member 5 is rotated in the hole 1a by about 180 degrees in the circumferential direction, as shown in Fig. 10B, the distal end portion of the supporting member 5 is restored to the bent shape, thus bending the bending portion 4.

A description will be given of how to use the supporting member 5. At the time of tissue incision, the bending portion 4 is formed into the straight shape shown in Fig. 10A, and, in the vicinity of the pericardium Y, the bending portion 4 is formed into the bent shape shown in Fig. 10B. Accordingly, the pericardium Y can be punctured by the blade tip 31a at a smaller angle to the heart Z. After the pericardium Y is punctured by the blade tip 31a, the supporting member 5 is pulled out from the hole 1a, and the distal end portion 3 is moved farther forward in the pericardial cavity X, in the same way as shown in Fig. 6 and Fig. 7.

In this modification, since the difference in width between the first notches 42 and the second notches 43 sets the bending direction of the bending portion 4, the distal end portion 3 may have a straight shape, as shown in Fig. 12.

### Third Modification

A puncture needle 1 according to a third modification of this embodiment is obtained by further modifying the second modification, and, as shown in Figs. 13A and 13B, the second notches 43 are omitted.

According to this modification, it is more difficult for the bending portion 4 to be bent in a direction in which the blade surface 31 is made to face outward, thus making it possible to more accurately set the bending direction of the bending portion 4. Although an arc-like distal end portion 3 is shown in Figs. 13A and 13B, the straight-shaped distal end portion 3 shown in Fig. 12 may be adopted.

Furthermore, in this modification, as shown in Fig. 14, it is possible to provide, instead of the supporting member 5, a wire (wire member) 6 that is located in the hole 1a along the axis-A direction, whose distal end is fixed at the distal end portion 3, and whose base end is located at the base end of the main body 2.

By doing so, when a base end portion of the wire 6 is pulled, the bending portion 4 can be bent at a desired timing and at a desired angle.

### Fourth Modification

In a puncture needle 1 according to a fourth modification of this embodiment, as shown in Fig. 15, the bending portion 4 has a first portion 44 (region indicated by hatching) that is located on the inner circumferential side and a second portion 45 that is located on the outer circumferential side, the first portion 44 and second portion 45 being made of flexible materials. The first portion 44 has a higher flexibility than the second portion 45, thereby constituting the bending-direction control means.

In this way, by making the bending portion 4 have different flexibilities in the circumferential direction, the flexural stiffness of the bending portion 4 in a bending direction in which the blade surface 31 is made to face inward is made higher than the flexural stiffness thereof in a bending direction in which the blade surface 31 is made to face outward, thus making it possible to set the bending direction of the bending portion 4.

The bending portion 4 is fabricated by joining a plurality of members that are formed of different materials or that have different thicknesses. Alternatively, the bending portion 4 may be configured such that the balance of materials is gradually changed in the circumferential direction, thereby gradually changing the flexibility in the circumferential direction.

### Fifth Modification

In a puncture needle 1 according to a fifth modification of this embodiment, as shown in Fig. 16, the bending portion 4 has an elastic member (for example, coil spring) 46 whose ends are connected to the distal end portion 3 and to the main body 2 and that is extendable/contractible in the axis-A direction and an extension restricting member 7 that restricts extension of the inner circumferential side of the elastic member 46 in the axis-A direction. In Fig. 16, the extension restricting member 7 connects, in the axis-A direction, turns of a spiral wire rod forming the coil spring, which is shown as an example of the elastic member 46, thereby restricting expansion of gaps between the turns of the wire rod in the axis-A direction.

In this way, by restricting extension of the elastic member 46 partially in the circumferential direction, the flexural stiffness of the bending portion 4 in a bending direction in which the blade surface 31 is made to face inward is made higher than the flexural stiffness thereof in a bending direction in which the blade surface 31 is made to face outward, thus making it possible to set the bending direction of the bending portion 4.

In this modification, as shown in Fig. 17, it is possible to adopt, as the extension restricting member 7, a member made of a material that has flexibility and that does not extend in the longitudinal direction, such as a wire, one end thereof being fixed to the distal end portion 3, and the other end thereof being fixed to the main body 2. With this configuration, the bending direction of the bending portion 4 can also be set.

### Sixth Modification

In a puncture needle 1 according to a sixth modification of this embodiment, as shown in Figs. 18A and 18B, the bending portion 4 is made of a flexible material and has, on the inner circumferential side, a thin-walled portion 47 whose wall thickness is thinner than that on the outer circumferential side.

In this way, by reducing the wall pressure of the elastic member 46 partially in the circumferential direction, the flexural stiffness of the bending portion 4 in a bending direction in which the blade surface 31 is made to face inward is made higher than the flexural stiffness thereof in a bending direction in which the blade surface 31 is made to face outward, thus making it possible to set the bending direction of the bending portion 4.

### Seventh Modification

In a puncture needle 1 according to a seventh modification of this embodiment, as shown in Fig. 19, the bending portion 4 has slits 48 that are spirally formed in a region that does not include an inner-circumferential-side portion.

In this way, by providing a region in which the slits 48 are not formed, partially in the circumferential direction, the flexural stiffness of the bending portion 4 in a bending direction in which the blade surface 31 is made to face inward is made higher than the flexural stiffness thereof in a bending direction in which the blade surface 31 is made to face outward, thus making it possible to set the bending direction of the bending portion 4. Furthermore, according to this modification, when the slits 48 are formed through laser processing, it is only necessary to control rotation of the bending portion 4 in the circumferential direction, movement of laser light in the axis-A direction, and the on/off state of the laser light, thereby making is possible to facilitate the processing.

As described above, in this embodiment and the modifications thereof, the blade surface 31, which is formed of the inclined distal end surface, is provided; however, instead of this, the distal end portion 3 may have a blade surface 31 and a blade tip 31a that are formed over the whole circumference of the distal end, as shown in Fig. 20.

With a puncture needle 1 having this blade surface 31, the bending portion 4 can be passively bent in the pericardial cavity X, thereby making it possible to prevent the blade tip 31a from being brought into contact with the heart Z.

### {Reference Signs List}

- 1: puncture needle
- 1a: hole
- 2: main body
- 3: distal end portion (bending-direction control means)
- 31: blade surface
- 31a: blade tip
- 31b: blade base
- 4: bending portion
- 41: bending segment
- 41a: axial portion
- 41b: notch portion
- 42: first notch (bending-direction control means)
- 43: second notch (bending-direction control means)
- 44: first portion (bending-direction control means)
- 45: second portion (bending-direction control means)
- 46: elastic member
- 47: thin-walled portion (bending-direction control means)
- 48: slit (bending-direction control means)
- 5: supporting member
- 6: wire
- 7: extension restricting member (bending-direction control means)
- 10: guide wire
- X: pericardial cavity
- Y: pericardium
- Z: heart

## Claims

1. A puncture needle comprising:
an elongated approximately-linear main body;
a distal end portion that is located at a distal end of the main body and that has a blade surface formed of a distal end surface which is formed inclined with respect to a longitudinal direction;
a bending portion that is provided between the main body and the distal end portion to connect the main body to the distal end portion and that can be passively bent in response to an external force; and
a bending-direction control means that sets a bending direction of the bending portion to a direction in which the blade surface is made to face toward an inner side of a bent shape.

2. A puncture needle according to claim 1, wherein the bending-direction control means is configured when the distal end portion is bent in an arc-like manner with the blade surface facing toward the inner side.

3. A puncture needle according to claim 1 or 2,
wherein the bending portion has a first flexural stiffness in a bending direction in which the blade surface is made to face toward the inner side and a second flexural stiffness in a bending direction in which the blade surface is made to face toward an outer side; and
the bending-direction control means is configured when the first flexural stiffness is smaller than the second flexural stiffness.

4. A puncture needle according to claim 3,
wherein the bending portion has notches that are formed on a lateral surface and that extend in a circumferential direction thereof; and
the notches are formed, in the circumferential direction of the bending portion, only on the same side as a blade base of the blade surface.

5. A puncture needle according to claim 3,
wherein the bending portion has notches that are formed on a lateral surface and that extend in a circumferential direction thereof; and
the notches are formed, in the circumferential direction of the bending portion, on the same side as a blade base of the blade surface and on the same side as a blade tip of the blade surface, and the notches that are formed on the same side as the blade base have larger widths in the longitudinal direction than the notches that are formed on the same side as the blade tip.

6. A puncture needle according to claim 3, wherein, in the circumferential direction of the bending portion, a side of the bending portion that is the same side as a blade base of the blade surface is made of a material having a higher flexibility than that of a side thereof that is the same side as a blade tip of the blade surface.

7. A puncture needle according to claim 3, wherein the bending portion comprises:
an elastic member that is extendable/contractible in the longitudinal direction of the main body; and
an extension restricting member that restricts extension of the elastic member, on the same side as a blade base of the blade surface in the circumferential direction of the elastic member.

8. A puncture needle according to claim 3, wherein the bending portion is formed of a cylindrical member and has a thin-walled portion having a thinner wall thickness on the same side as a blade base of the blade surface in the circumferential direction than on the same side as a blade tip of the blade surface.

9. A puncture needle according to claim 3, wherein the bending portion is formed of a cylindrical member and has spiral slits formed in the circumferential direction in a region that excludes the same side of a blade base of the blade surface.

10. A puncture needle according to one of claims 1 to 9,
wherein the main body, the bending portion, and the distal end portion are formed of a cylindrical member that has, therein, a hole passing therethrough in the longitudinal direction; and
the puncture needle further comprises an approximately-linear supporting member that is inserted into the hole of the main body, the bending portion, and the distal end portion, which are communicated with each other in the longitudinal direction, and that has a larger stiffness than the bending portion.

11. A puncture needle according to claim 10, wherein a distal end portion of the supporting member has a bent shape and can be elastically deformed into an approximately-linear shape.

12. A puncture needle according to one of claims 1 to 9, further comprising a wire member whose distal end is connected to the distal end portion and that extends to the base end of the main body.
